Europäisches Patentamt

European Patent Office

Office européen des brevets

(11) Publication number: **0 348 005**
**A1**

# (12) EUROPEAN PATENT APPLICATION

(21) Application number: **89201636.1**

(51) Int. Cl.⁴: **A61F 2/16**

(22) Date of filing: **20.06.89**

The title of the invention has been amended (Guidelines for Examination in the EPO, A-III, 7.3).

(30) Priority: **20.06.88 NL 8801572**

(43) Date of publication of application:
**27.12.89 Bulletin 89/52**

(84) Designated Contracting States:
**AT BE CH DE ES FR GB GR IT LI LU NL SE**

(71) Applicant: **EYETECH ADVIES B.V.**
**Borchwerf 14**
**NL-4704 RG Roosendaal(NL)**

(72) Inventor: **Van Endt, Johannes Jacobus**
**Euklaasdijk 18**
**NL-4706 JA Roosendaal(NL)**

(74) Representative: **Hoorweg, Petrus Nicolaas et al**
**OCTROOIBUREAU ARNOLD & SIEDSMA**
**Sweelinckplein**
**NL-2517 GK The Hague(NL)**

(54) **Intraocular lens.**

(57) The invention relates to a round lens-shaped element (1) as replacement for the natural lens in the human or animal eye, whereby the posterior or support surface has a relatively small radius of curvature corresponding with the natural bulging of the lens and the anterior surface (3) in the central lens-shaped optical portion (2) of the element is provided with an annular edge portion (5) of the same or virtually the same thickness.

This lens provides a prothesis whereby the eye tissues are supported in sufficient measure so that no further deformation can occur.

The edge portion preferably extends in the line of the posterior surface of the central lens-shaped portion.

FIG.6

## Lens Element

The invention relates to a round lens-shaped element as replacement for the natural lens in the human or animal eye.

In the case of lens deficiencies, particularly the turbid lens caused by cataracts, it is necessary to remove the lens from the eye, following which in accordance with the known art a lens of transparent material is positioned to replace the natural cortex and nucleus of the lens. A known lens as according to GB-2151371 has a comparatively large size and is attached and placed under tension. Such a lens has the drawback that the eye tissues are not supported to a sufficient extent, as a result of which deformation of the eye, namely the vitreous body, can occur. In any case the known eye chamber lenses are fixed in the lens capsule and are the cause of fold formation in the capsule. Fixation in the capsule moreover leads, if it creases, to further displacement of the lens implant with the consequent decentering of the lens.

The invention has for its object to provide a lens replacement element, whereby the above stated drawbacks do not occur. To this end the element according to the invention is distinguished in that the posterior or support surface has a relatively small radius of curvature corresponding with the convex form of the natural lens, and the anterior surface in the central lens-shaped optical portion of the element is provided with an annular edge part of the same or virtually the same thickness.

Because of the edge part the vitreous body is on the one hand again supported, in contrast to other artificial lenses, as a result of correct fit, with the result that less traction of the vitreous body on the suspension points of the retina will occur (with less chance therefore of retinal detachment).

On the other hand this natural convex form gives a better positioning and centering of the invention in the natural shape of the capsule relative to the other artificial lenses, as the capsule is not used as primary fixation of the invention but as initial insertion place.

The lens capsule, from which the old lens is removed and in which the invention is placed, is also stretched into position again in a natural manner, without wrinkles.

Above mentioned and other features of the invention will be elucidated in the description of a number of embodiments following below.

In the annexed drawing:
Fig. 1 shows a perspective rear view of an element according to the invention,
fig. 2,3 and 4 each show a standing cross section of alternative embodiments of the element according to the invention,

fig. 5 is a rear view of the element,
fig. 6 is a section whereby the element is placed in the eye.

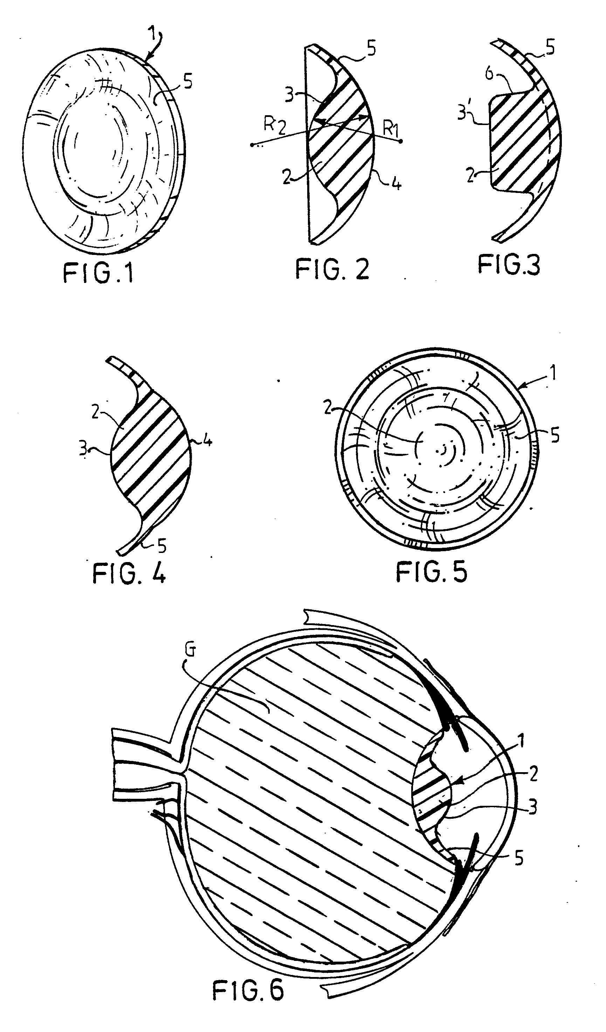

Indicated in the figures with the numeral 1 is the lens element which as according to fig. 5 has a circular shape as seen in the principal optic axis and which displays a constant thickness over the edge portion of for instance 0.2 mm. The diameter hereby varies between 7 and 10 mm, and is preferably 9 mm.

The central portion 2 of the element preferably takes a biconcave form, but may nevertheless have different forms.

Thus for example the lens-shaped part 2 in fig. 2 has an anterior surface 3 which has a smooth curving form and which is curved in accordance with a determined radius of curvature R1. The posterior surface 4 is curved in accordance with a determined radius of curvature R2. The posterior surface 4 of the lens-shaped central portion 2 preferably lies in the same spherical plane as the edge strip 5 but can at variance thereto have another radius of curvature as in fig. 4.

The variation of the radius of curvature R2 can lie between 4.5 and 13 mm, and is preferably 6 mm.

The variation of the radius of curvature R1 is such that a lens power is obtained ranging from -50 to +40 dioptre.

Fig. 3 shows in contrast that the central lens portion 2 has a flat anterior side 3′ which transposes via a bent part 6 into the anterior surface of the edge portion 5.

It is of course also possible to embody the radius of curvature R1 such that a multi-focal lens system is obtained, which results in it being possible to develop a different depth of focus.

Fig. 6 shows how the lens element 1 is implanted in the eye. To this end, after the natural lens has been removed by the known eye operation, the edge portion 5 will be placed beneath the iris of the eye in the lens capsule such that the central lens portion 2 comes to lie precisely on the location of the natural lens. Since the natural lens has a determined natural curvature on the posterior side 4, the posterior surface 4 of the lens 2 is adapted to this curvature so that the posterior surface 4 connects precisely onto the vitreous body G of the eyeball. In this way the vitreous body is supported by the posterior side 4 of the lens and no deformation of the tissue will occur. On the other hand the lens 2 is held accurately in position in the lens capsule because of the curvature of the surface 4, as a result of which further fixation is unnecessary.

The invention is not limited to the above described embodiments.

## Claims

1. Round lens-shaped element as replacement for the natural lens in the human or animal eye, **characterized in that** the posterior or support surface has a relatively small radius of curvature corresponding with the natural bulging of the lens and the anterior surface in the central lens-shaped optical portion (2) of the element (1) is provided with an annular edge portion (5) of the same or virtually the same thickness.

2. Element as claimed in claim 1, **characterized in that** the edge portion extends in the line of the posterior surface (4) of the central lens-shaped portion (2).

3. Element as claimed in claims 1 and 2, **characterized in that** the central portion (2) and the edge portion (5) have the same radius of curvature.

4. Element as claimed in any of the foregoing claims, **characterized in that** the radius of curvature of the anterior surface (3) of the lens is the same as that of the posterior surface (4).

5. Element as claimed in any of the foregoing claims, **characterized in that** the radius of curvature of the anterior surface of the lens-shaped portion (2) deviates from that of the posterior surface of that portion.

6. Element as claimed in any of the foregoing claims, **characterized in that** the radius of curvature of the anterior surface (3) of the lens-shaped central portion (2) varies from place to place.

7. Element as claimed in any of the foregoing claims, **characterized in that** the radius of curvature of the outer surface lies between 4.5 and 13 mm.

8. Element as claimed in any of the foregoing claims, **characterized in that** the diameter of the described circle as seen in the principal optic axis is no more than 9 mm.

FIG.1

FIG. 2

FIG.3

FIG. 4

FIG. 5

FIG.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 136 807 (EZEKIEL NOMINEES) <br> * Figures; page 5, lines 23-24; page 11, line 22 * | 1-5 | A 61 F 2/16 |
| Y | | 6 | |
| A | | 7,8 | |
| | --- | | |
| Y | WO-A-8 603 961 (VANNAS) <br> * Claim 7; abstract; figures * | 6 | |
| | --- | | |
| D,X | GB-A-2 151 371 (WICHTERLE) <br> * Abstract; page 2, lines 24-48; figures * | 1-3,5 | |
| | --- | | |
| A | FR-A-2 530 457 (MAZZOCCO) | | |
| | --- | | |
| A | US-A-3 711 870 (DEITRICK) | | |
| | ----- | | |

TECHNICAL FIELDS
SEARCHED (Int. Cl.4)

A 61 F

The present search report has been drawn up for all claims

| Place of search | Date of completion of the search | Examiner |
|---|---|---|
| THE HAGUE | 21-09-1989 | STEENBAKKER J. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P0401)